(19) Europäisches Patentamt
European Patent Office
Office européen des brevets

(11) **EP 3 460 808 A1**

(12) **EUROPEAN PATENT APPLICATION**

(43) Date of publication:
**27.03.2019 Bulletin 2019/13**

(51) Int Cl.:
**G16H 50/70** (2018.01)

(21) Application number: **17192399.8**

(22) Date of filing: **21.09.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **DEN TEULING, Nicolaas Gregorius Petrus
5656 AE Eindhoven (NL)**
• **PAUWS, Steffen Clarence
5656 AE Eindhoven (NL)**
• **KLEE, Mareike
5656 AE Eindhoven (NL)**

(74) Representative: **de Haan, Poul Erik
Philips International B.V.
Philips Intellectual Property & Standards
High Tech Campus 5
5656 AE Eindhoven (NL)**

(54) **DETERMINING PATIENT STATUS BASED ON MEASURABLE MEDICAL CHARACTERISTICS**

(57) A method of generating an algorithm for determining a condition of a patient. The algorithm is generated based on medical characteristics that are shared by a group of data sources. The group of data sources is obtained by clustering a plurality of data sources into groups based on the medical characteristics associated with each data source.

FIG. 1

EP 3 460 808 A1

**Description**

FIELD OF THE INVENTION

This invention relates to algorithms for determining a patient status based on measurable medical characteristics.

BACKGROUND OF THE INVENTION

Remote health monitoring services, also named telehealth services, provide the opportunity for chronically ill patients to be monitored and managed at home. Algorithms are used to detect when the condition or status of a patient changes. This detection may be used to raise an alarm when a patient status begins to deteriorate, and thereby reduces the number of emergency admissions, caused by missing a deterioration of a patient, and improves overall patient outcome and quality of life.

The utilization of algorithms for remotely monitoring patient status is a relatively new area of research. These algorithms use medical characteristics of a patient (such as body weight, heart rate, breath/respiratory rate, blood pressure, surveys, activity and so on) to determine a patient status. Medical characteristics are typically obtained by patient monitoring devices, such as pulse rate monitors or respiratory rate monitors.

There is an increasing desire to use remote health monitoring services in new settings, regions and locations, which may use a variety of different monitoring devices. Similarly, there is an increasing desire to improve optimization of algorithms for determining a patient status, in order to improve recognition of patient status and thereby avoid potentially missing a deterioration of a patient.

SUMMARY OF THE INVENTION

The invention is defined by the claims.

According to examples in accordance with an aspect of the invention, there is provided a method of generating at least one algorithm for determining a status of a patient, the method comprising: receiving input data which indicates measurable medical characteristics for each of a plurality of data sources; clustering, using the input data, the data sources into one or more groups based on the measurable medical characteristic for each of the data sources; and calculating, for each of one or more groups of data sources, an algorithm for determining a status of a patient based on the measurable medical characteristics for the respective group of data sources.

The inventive concept provides a method of grouping, clustering or arranging data sources into clusters or groups, and generating an algorithm for determining a patient status or status for each of a selection of these groups. Groups are clustered based on the measurable medical characteristics associated with the data sources, such that data sources that share the same measurable medical characteristics are generally arranged into a same group.

A measureable medical characteristic is commonly referred to as a sign, symptom or observation of a patient. A patient monitoring device is able to monitor various medical characteristics of a patient using a variety of different possible inputs. Thus a patient monitoring device may be associated with particular measurable medical characteristics. A monitoring device may be an automated monitoring device, such as a pulse rate monitor, or a manually controlled monitoring device, such as a clinician reporting tool for recording observations or surveys.

Similarly, a patient may be associated with various measurable medical characteristics, being characteristics of that patient which are monitored by a patient monitoring device.

Thus, there are at least two potential data sources which identify the measurable medical characteristics of a given patient, being the patient himself or a patient monitoring device. Other data sources include diagnoses (which are commonly associated with desired medical characteristics for measurement) and treatment options (which may define medical characteristics to be monitored to ensure adherence to the treatment and/or a reaction of a patient to a treatment option).

Algorithms can thereby be optimized using at least a patient-based analysis, a device-based analysis, a diagnoses-based analysis and/or treatment-based analysis.

The method allows algorithms for determining a patient status to be optimized/improved without the need to calculate a specific (i.e. individualized) algorithm for a particular data source (such as a device or patient). This provides significant improvements as to an accuracy of determining a patient condition/status whilst minimizing an amount of processing required to generate an algorithm. In particular, algorithms suitable for and/or optimized for a plurality of patient data sources may be generated thereby avoiding the need to repeatedly calculate an algorithm for individual patient monitoring devices.

An improved accuracy in determining a patient status reduces a likelihood of missing a deteriorating patient. Thus, patient outcomes are improved as deterioration of a patient is more accurately recognized.

As each group is clustered based on the available measurable medical characteristics, data sources in a group may share measurable medical characteristics. This allows an algorithm for determining a patient status to be tuned to a common set of measurable medical characteristics. In particular, a best compromise algorithm may be generated for data sources in a same group.

The input data may be obtained from example patient data (which would identify what medical characteristics of a patient are being monitored) or from a list of available inputs for the plurality of monitoring devices.

The invention may be, for example, be used to modify a known algorithm for a new site with different data sources (i.e. than the original algorithm). For example, when the data sources represent patient monitoring devices, this would enable one or more known algorithms to be optimized for monitoring devices of a new site with increased efficiency and accuracy, as the monitoring devices may be grouped effectively.

The invention may be employed, for example, when a patient changes location, hospital or region, which would affect which medical characteristics of that patient can be monitored. When a patient changes location, the plurality of devices associated with that location also changes, adjusting the possible available patient data inputs for that patient. Thus, an algorithm for determining the patient status will benefit from altering for a new set of data sources.

In other embodiments, the invention may be employed when a new diagnosis is made or a new treatment option is considered. This will allow data sources to be grouped appropriately to consider the best option available to a clinician.

Each data source may represent one of: a patient; a patient monitoring device; a diagnosis; and a treatment option. Thus a data source may represent a grouping of medical characteristics, each grouping being associated with a particular device, patient or medical consideration (e.g. diagnosis or treatment option).

In some embodiments, the input data further indicates, for each of the data sources, a importance value of each measurable medical characteristic associated with said data source; and the clustering, using the input data, is performed based on the importance values of the measurable medical characteristics associated with each of the data sources.

Thus a relative importance of each measurable medical characteristic to a data source may be used to more optimally cluster medical devices. For example, medical data sources sharing a same (relatively important) measurable medical characteristic may be grouped.

Embodiments thereby enable algorithms for calculating a patient status to be designed or optimized for groups in which an importance of a measurable medical characteristic is considered. Thus, algorithms may be specifically designed for groups which substantially share a same measurable medical characteristic of high relative importance, and thereby provide a more optimal algorithm for determining a status/condition of a patient, as importance characteristics are taken into account for determining a patient status.

Relative importance could be a relative importance to an algorithm for determining a status of one or more patients (e.g. a pulse rate may be of more importance for determining a status than a body weight), a relative importance to the medical device (e.g. an importance being representative of intended purpose or accuracy of the monitoring device) or a relative importance of a diagnosis/treatment option (e.g. a heart rate may be more important when treating a heart condition).

In one example, where the input data represents patient monitoring devices, a pulse oximeter (patient monitoring device) may be able to record both SpO2 and pulse rate. However, the SpO2 may be of importance for certain algorithms for determining a patient status, and thus may have a high relative importance for an algorithm. Similarly, the primary purpose of a pulse oximeter is to provide SpO2 readings, such that a relative importance to the medical device may be high. It may therefore be advantageous to priorities grouping the pulse oximeter with devices which share a capability of measuring SpO2 (rather than heart rate), so that a calculated algorithm for that group may use this medical characteristic to best advantage.

Such methods generate a more accurate algorithm for determining a patient status.

In some embodiments, the input data further indicates, for each data source, a temporal availability of each measurable medical characteristic associated with said data source; and the clustering, using the input data, is performed based on the temporal availability of the measurable medical characteristics associated with each of the data sources.

A temporal availability of a measurable medical characteristic indicates how often, regularly or reliably a measurable medical characteristic may be obtained by a particular monitoring device or with respect to a patient. This information may be used to group, for example, devices which record at a similar speed or regularity or patients which are recorded at a similar speed or regularity. Thus, algorithms generated for such groups may take into account a likely speed of data gathering or a regularity of a medical characteristic being measured or sampled. A generated algorithm may be less resource/time intensive if a regularity of data gathering for a group is high, and may be more resource-time intensive if a regulatory of data gathering for a group is low. This allows for more customization and a reduction in processing power.

The step of clustering may comprise: applying a plurality of different clustering algorithms to the input data to obtain a respective plurality of clustering results, each clustering result comprising all of the data sources clustered into one or more groups; calculating a coverage value for each clustering result, the coverage value indicating a percentage of data sources meeting a predetermined criterion; and selecting a clustering result based on the coverage value of each clustering result.

A method may therefore comprise performing numerous clustering operations for a variety of scenarios (e.g. using different clustering methods or using a same clustering producing different numbers of groups). A coverage value of each scenario (clustering result) may be used to determine which clustering algorithm has provided an appropriate clustering of the data sources.

The above-described embodiment thereby allows for more accurate clustering of data sources, which results in improve optimization of algorithms for determining a patient status.

The selecting the clustering result may comprise selecting the clustering result which is associated with one or more of: the greatest coverage value; the greatest value resulting from dividing the coverage value by the number of groups in the clustering result; and the clustering result having the lowest number of groups amongst the clustering results having a coverage value greater than a predetermined coverage value.

Thus, it may be assessed how well the groups of each clustering result apply to the data sources and/or algorithms. This may significantly improve a grouping or clustering of the data sources (i.e. provide the most appropriate groupings) and thereby provide a more accurate and optimized algorithm for determining a patient status.

The predetermined coverage value may be determined, provided or set by a user of the method, but is preferably greater than 50%, for example, greater than 70%, for example greater than 80% or 90%. A coverage value in the region of 80% has been identified as providing suitable values.

In some embodiments, the data source meets the predetermined criterion if the measurable medical characteristics for said data source comprise the most common measurable medical characteristic of all data sources in the same group of the said data source.

This ensures that groups of data sources are generally associated with at least one same measurable medical characteristic, and thereby ensures that an accuracy and appropriateness of an algorithm generated for the group of monitoring devices is improved. For example, an algorithm for a group containing a same common measurable medical characteristic may be optimized for determining a patient condition based on that same measurable medical characteristic (across a wide variety of devices).

In at least one embodiment, the input data further indicates, for each data source, a importance value of each measurable medical characteristic associated with said data source; and a data source meets the predetermined criterion if at least one available measurable medical characteristics for said data source which has a importance value equal to or above a predetermined importance value is common to more than a predetermined proportion of the data sources in the same group as the said data source

Thus, methods may ensure that data sources are grouped based on an importance of measurable medical characteristic, such that data sources having a same important measurable medical characteristic may be generally grouped together. This ensures that an accuracy and appropriateness of an algorithm generated for the group of data sources is improved.

The method may be adapted wherein the input data further indicates, for each data source, a importance value of each measurable medical characteristic associated with said data source; and a data source meets the predetermined criterion if all measurable medical characteristics for the said data source, which have a importance value equal to or above a predetermined importance value, are common to more than a predetermined proportion of the measurable medical characteristics in the same group as the said data source.

In at least one embodiment, the clustering comprises: applying a plurality of different clustering algorithms to the input data to obtain a respective plurality of clustering results, each clustering result comprising all of the data sources clustered into one or more groups; calculating a distinctiveness score for each clustering result, the distinctiveness score indicating a distinctiveness of the groups within a respective clustering result; selecting a clustering result based on the distinctiveness score of each clustering result.

The calculated algorithm for a group of data sources may be based on at least the most common measurable medical characteristic within the group of data sources.

By identifying the most common set of co-occurring medical characteristic, an algorithm may be readily optimized for a particular group of data sources. This would improve an accuracy of identifying a patient status.

According to another aspect of the inventive concept, there is proposed a computer program product comprising a computer readable storage medium having computer readable program instructions embodied therewith to, when executed on a processor arrangement, cause said processor arrangement to implement any method previously described.

According to yet another aspect of the inventive concept, there is proposed a processor arrangement for generating at least one algorithm for determining a status of one or more patients, the processor arrangement comprising: a data receiving unit adapted to receive input data which indicates measurable medical characteristics for each of a plurality of data sources; a clustering unit adapted to cluster, using the input data, the data sources into at one or more based on the measurable medical characteristic for each of the data sources; and a calculating unit adapted to calculate, for each of one or more groups of data sources, an algorithm for determining a status of a patient based on the measurable medical characteristics for the respective group of data sources.

In at least one embodiment, the input data further indicates, for each of the data sources, a importance value of each measurable medical characteristic associated with said data source, and the clustering unit is adapted to cluster, using the input data, is performed based on the importance values of the measurable medical characteristics associated with each of the data sources.

In some embodiments, the input data indicates, for each data source, a temporal availability of each measurable medical characteristic associated with said data source; and the clustering unit is adapted to cluster, using the input data, is performed based on the temporal availability of the measurable medical characteristics associated with each of the data sources.

The clustering unit may be adapted to apply a plurality of different clustering algorithms to the input data to obtain a respective plurality of clustering results, each clustering result comprising all of the data sources clustered into one or more groups; calculate a coverage value for each clustering result, the coverage value indicating a percentage of data sources meeting a predetermined criterion; select a clustering result based on the coverage value of each clustering result.

BRIEF DESCRIPTION OF THE DRAWINGS

Examples of the invention will now be described in detail with reference to the accompanying drawings, in which:

Figure 1 shows a method of generating an algorithm according to an embodiment of the invention;
Figure 2 illustrates a clustering result during a method of generating the algorithm according to an embodiment of the invention;
Figure 3 illustrates a step of clustering data sources according to an embodiment of the invention; and
Figure 4 illustrates a processor arrangement for generating an algorithm according to an embodiment of the invention.

DETAILED DESCRIPTION OF THE EMBODIMENTS

According to a concept of the invention, there is proposed a method of generating an algorithm for determining a condition of a patient. The algorithm is generated based on medical characteristics that are shared by a group of data sources. The group of data sources is obtained by clustering a plurality of data sources into groups based on the medical characteristics associated with each data source.

Embodiments are at least partly based on the realization that algorithms for determining a status of a patient can be optimized for a large group of data sources simultaneously. This would reduce a processing power required to optimize an algorithm for a particular data source (as optimization is not performed on a source-by-source basis) whilst enabling improvements and adaptations to an algorithm to be effected.

Illustrative embodiments may, for example, be employed in telehealth solutions where a patient is remotely monitored. Such solutions benefit from algorithms for automatically determining a patient status from medical characteristics (e.g. with reduced clinician input) to identify if a patient status deteriorates.

It has previously been described how algorithms may be used to determine a status, condition or health of a patient based on measurable medical characteristics of a patient. The measurable medical characteristics represent signs, symptoms or other medical observations of the patient. In particular, the measurable medical characteristics may be a scalar, categorical or binary value indicating a magnitude/presence of a particular sign or symptom of a patient.

In one example, an algorithm may determine that a status of a patient has deteriorated if the patient's heart rate falls outside a predetermined acceptable range or a blood sugar level falls below a predetermined value.

A typical approach to identifying a patient status is to use logistic regression based on a number of possible characteristics measured quantitatively or numerically. By way of example, one possible algorithm based on a patient's weight (w), (systolic or diastolic) blood pressure (BP), heart rate (HR) and oxygen saturation ($SpO_2$) may be as follows:

$$logit(p) = b_0 + b_1.w + b_2.BP + b_3.HR + b_4.SpO_2 \qquad (1)$$

where p represents a probability of deterioration, $b_0$ represents a baseline risk and coefficients $b_1$ - $b_4$ represent a relative influence of a patient characteristic on the probability of deterioration.

The probability of deterioration p may then be calculated using the following standard well known equation:

$$p = \frac{1}{(1 + e^{-logit(p)})} \qquad (2)$$

Other embodiments for algorithms may instead use logical or descriptive patient characteristics to advantage, or convert logical or descriptive medical characteristics (such as skin color or area of pain) to appropriate numerical values. Thus, descriptive or logical values may be converted to quantitative or numerical values.

It is therefore clear that algorithms advantageously provide autonomous methods of identifying a patient status, such a probability of a patient's deterioration. This reduces a need for clinician presence or input to determine a condition of a patient, thereby enabling a patient to be more consistently monitored. Thus, a deterioration of a patient is less likely to be missed and patient outcome is significantly improved.

A data source is used to identify groups of measurable medical characteristics that are or could be associated with a particular (hypothetical or real) patient or patient monitoring device. The data source may be otherwise labelled a medical data source, an input source or an input vector.

A data source may, for example, represent a patient, a patient monitoring device, a diagnosis or a treatment option. Each of these data sources is associated with particular measurable medical characteristics.

In one example, it may be desirable, known or commonplace, for a particular diagnosis or when conducting a particular treatment option, to monitor a minimum set of measurable medical characteristics. Thus, a diagnosis or treatment option may be associated with a group of measurable medical characteristics, and may thereby be represented by a data source. For example, it may be desirable, when treating a heart condition, to monitor at least a pulse rate and blood pressure.

In another example, a patient may be monitored by various monitoring devices recording medical characteristics of the patient. Thus, the patient is associated with measurable medical characteristics (being all the characteristics measured by monitoring devices monitoring the patient). Thus, a data source may represent a patient.

In yet another example, a patient monitoring device monitors a defined set of measurable medical characteristics of a (hypothetical) patient. The patient monitoring device may thereby be itself considered as a data source, associated with measurable medical characteristics, being those characteristics that the patient monitoring device may monitor.

It is therefore clear that a wide variety of different data sources may be associated with measurable medical characteristics. Algorithms for monitoring a patient status may be employed from the perspective or 'level' of any one of these different types of data source.

At a 'patient level', an algorithm may identify an overall status of a particular patient, and may be capable of using any of the available measurable medical characteristics of the patient (e.g. from a plurality of different monitoring devices).

At a 'device level', an algorithm may identify a status of a patient from the perspective of a particular monitoring device, so that the algorithm can only use the available medical characteristics associated with the monitoring device.

At a 'treatment level', an algorithm may look to determine a status of a patient using the measurable medical characteristics associated with that treatment.

Thus, each data source may be associated with a measurable medical characteristic and an algorithm for determining a status of the patient based on these measurable medical characteristics.

Consider a scenario in which a patient is monitored by a first monitoring device monitoring a patient's heart rate and blood pressure, and a second monitoring device monitoring a patient's respiratory rate and respiratory volume. The patient is therefore associated with four measureable medical characteristics.

A first algorithm may be performed at a 'patient level' to determine an overall status of the patient, and may use all four measurable medical characteristics.

A second, different algorithm may be performed at a 'device level' to determine a status of the patient as monitored by a particular device. For example, the first monitoring device may run the second algorithm, and only be able to use the measurable medical characteristics associated with the first monitoring device.

Performing an algorithm at a 'patient level' enables a more accurate and complete analysis of a patient status to be performed, as the medical characteristics monitored by multiple monitoring devices may be taken into account. Patient level algorithms may mitigate against potential failures or errors in monitoring devices.

Performing an algorithm at a 'device level' may enable each monitoring device to independently alert when a patient status has changed, potentially providing redundancy. Device level algorithms may allow for more specific analysis of a particular patient condition or area of diagnosis (e.g. heart-related diagnoses), as individual devices are commonly associated with a particular area of the patient (e.g. heart, lungs, blood etc.).

Performing an algorithm at a 'treatment level' enables a consistent approach to assessing patient status when performing a same treatment. This enables simpler analysis of treatment efficacy, as the treatment level algorithm may act as a control variable so that trends or similarities for treatment outcomes can be identified with a higher degree of accuracy and validity. This may thereby provide improvements to overall patient outcome.

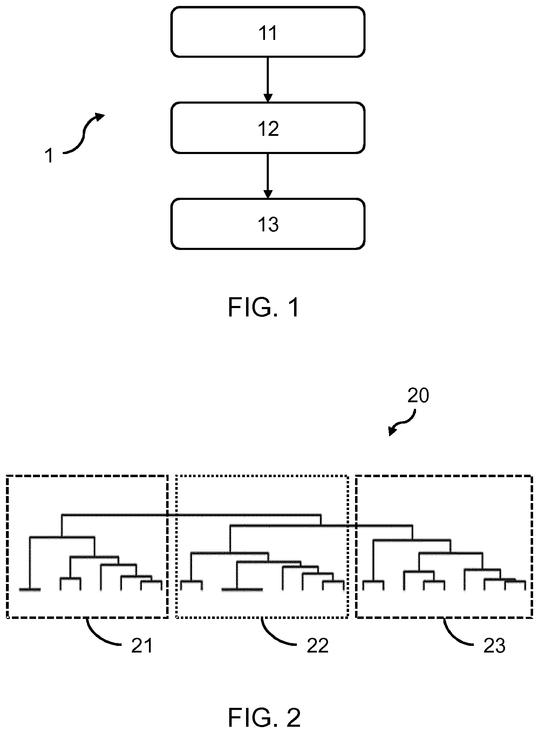

Figure 1 illustrates a method 1 of generating at least one algorithm according to a general embodiment of the invention.

The method comprises a step 11 of obtaining or receiving input data which indicates measurable medical characteristics associated with each of a plurality of data sources. For example, the input data may indicate an availability of measurable medical characteristics.

The input data, for example, may be a dataset comprising a plurality of records. Each record may be representative of a respective data source. The record may identify measurable medical characteristics associated with the data source.

In a first example, where each data source represents a patient, the input data may comprise patient records which indicate what medical characteristics (signs or symptoms) of that patient can be monitored. In one example, the input data comprises a first dataset, which identifies which patient monitoring devices are associated with a particular patient, and a second dataset which identifies capabilities of said patient monitoring devices.

In a second example, where each data source represents a patient monitoring device, the input data may comprise device records which indicating what medical characteristics each patient monitoring device is able to record.

In a third example, where each data source represents a treatment option or a potential diagnosis, the input data may comprise treatment records indicating what medical characteristics should be monitored when a (hypothetical) patient is undergoing a particular treatment option or has been provided with or assigned the potential diagnosis.

The method further comprises a step 12 of clustering, grouping or otherwise arranging the data sources into one or more groups, sets or clusters based on the measurable medical characteristics associated with each data source. Preferably, the step of clustering identifies at least two sets, groups or clusters of data source.

In particular, data sources associated with similar measurable medical characteristics may be grouped or arranged into the same groups so that a plurality of groups is identified from the input data.

In one example, the step of clustering comprises determining a level of similarity between the different data sources with respect to their medical characteristics, and grouping data sources having a level of similarity above a predetermined level. This may be performed according to any known algorithm, such as a clustering algorithm or a distance calculation algorithm, as described below.

The method 1 further comprises a step 13 of calculating, for at least one group, an algorithm for determining a status of a patient based on the measurable medical characteristics for the respective group of data sources. In preferable embodiments, an algorithm is tuned to a common set of medical characteristics of the at least one group.

Preferably, an algorithm is generated for every group of data sources, such that each data source can be associated with an algorithm, which is shared by the group. This would ensure that optimized algorithms are provided for a larger number of data sources, and thereby provide improved patient outcome.

The algorithm may be generated by modifying an existing, default or historical algorithm to reflect the medical characteristics common to a particular group, set or cluster of data sources. In one example, coefficients of an existing, default or historical algorithm may be adjusted to thereby tune or adapt the algorithm to the particular group of data sources. This may, for example, be performed using a technique such as dynamic model averaging, which is one focus of the area of research labelled transfer learning. Other techniques would be readily apparent to the skilled person, such as adapting or utilizing other approaches from the transfer learning research field.

In another example, certain variables of an algorithm can be removed based on the medical characteristics of the group (e.g. remove those variables associated with medical characteristics not shared by the group). Thus, an algorithm may be retrained or recreated using only the available measured characteristics of a group.

In other embodiments, the algorithm for a group of data sources may be calculated based on an average set of medical characteristics of the group of data sources (e.g. an average input vector of the group of data sources).

In this way, an algorithm for determining a patient status may be tuned to better represent the needs or capabilities of a particular group or cluster.

An algorithm for determining patient status may thereby be simultaneously generated for a plurality of data sources (i.e. each data source in a group). This allows for customization and optimization of an algorithm without the need for resource-intensive processing or calculation of individualized algorithms. In particular, a "best compromise" or "best-fit" algorithm may be generated for data sources lying a same group.

Optimizing an algorithm for a particular group ensures that at least the availability of particular medical characteristics is taken into account when determining an algorithm for determining a status of a patient. This improves an accuracy of the algorithm and better optimizes the algorithm for the purposes of data sources in the group as a whole.

Using a same algorithm for a plurality of data sources further provides a more consistent approach to assessing patient status across different data source (e.g. patients, monitoring devices, treatment options, diagnoses and so on). This will provide improved and more reliable prediction of patient outcome, as patients will be monitored in a same, optimized manner.

The method may be performed when a data source or input data is modified. For example, if a patient has been moved to a new site (thereby affecting the patient data of that new site), or when a device has been added to a bank of monitoring devices or when a new treatment option has become available. Such movement may cause the optimal algorithm for an overall group of patients to change or alter.

The invention thereby identifies frequently co-occurring medical characteristics by forming groups. This assessment can be made from example patient data (i.e. input data indicating a plurality of patients), a list of input types available per device (i.e. input data indicating a plurality of patient monitoring devices), or a list of diagnoses or treatment options which identifies medical characteristics to be monitored for a particular diagnosis or treatment option (i.e. the input data indicates a plurality of diagnoses or treatment options). A clustering algorithm is used to group the different input sets or sources into categories of frequently co-occurring inputs.

In embodiments, each data source may be represented by a respective data source vector Ps, which may act as a record of that data source:

$$P_s = (p_1, p_2 \ldots p_N) \qquad (3)$$

where p represents a (logical) availability of a different medical characteristic and N is a number of medical characteristics associated with the data source.

The availability of a medical characteristic may be represented by a logical/binary value or, as explained below, by a scalar value representing a temporal or historic availability.

The clustering performed in step 12 may be performed according to any known clustering method, for example, an agglomerative hierarchical cluster algorithm. This cluster algorithm uses a bottom-up approach to join similar input vectors into groups. In some embodiments, a distance between data sources (or input vectors) is calculated by a linkage criterion, e.g. by performing a Ward's minimum variance method.

Other clustering methods include a centroid model (e.g. k-means or k-medoids clustering, such as the Partitioning Around Medoids algorithm), a distribution model, a subspace model (e.g. biclustering) and so on. The similarity between input vectors (i.e. data sources) can be assessed using the Manhattan distance or by a weighted sum of differences. In one example, a Euclidean distance or a weighted Euclidean distance between different input vectors is calculated to determine similarities between data sources.

Table 1

| Medical Characteristic | A | B | C | D |
|---|---|---|---|---|
| Device 1 | Yes | Yes | No | No |
| Device 2 | Yes | Yes | No | No |
| Device 3 | Yes | Yes | No | No |
| Device 4 | Yes | No | Yes | Yes |
| Device 5 | Yes | No | Yes | Yes |
| Device 6 | Yes | No | Yes | Yes |
| Device 7 | No | No | Yes | Yes |
| Device 8 | No | No | Yes | Yes |

Table 1 illustrates a first use case scenario where the input data indicates medical characteristics (A-D) associated with a plurality of patient monitoring devices (i.e. data sources). In particular, there are eight patient monitoring devices (Devices 1-8), each able to monitor (i.e. associated with) particular medical characteristics of a patient (A-D).

The eight patient monitoring devices could be clustered into two sets or groups. A first group (Devices 1-3) associated with characteristics A, B (and not C, D); and a second group (Devices 4-6) associated with characteristics C, D (and not B). The grouped monitoring devices share at least one common medical characteristic with others monitoring devices in their respective groups.

Thus, a first algorithm for determining a patient status could be calculated for the first group on the basis of characteristics A and B (being the characteristics common to the first group); and a second algorithm could be calculated on the basis of characteristics C and D (being the characteristics common to the second group).

Figure 2 illustrates a clustering result 20 for a clustering algorithm performed on a different set of representative input data. In particular, Figure 2 provides a visual representation of the clustering result in the form of a dendrogram.

The representative input data represents medical characteristics for patient monitoring devices for monitoring sleep apnea patients at a new site. Twenty-six different device types are used, with a total of fifty-three unique inputs. Each device type is represented as a different data source.

Different monitoring devices can have a similar treatment purpose but have different features or monitoring capabilities due to vendor specification, product families, treatment options (such as CPAP, BiPAP, APAP machines), product generational differences and different vendors. Thus, each monitoring device may be associated with a different set of measurable medical characteristics. Using a hierarchical clustering method, it is possible to identify the underlying structure that groups or arranges the monitoring devices together.

The clustering result 20, performed on the basis of a hierarchical clustering method, shows that the twenty-six monitoring devices may be broadly divided or clustered into three groups 21, 22 and 23.

A clustering algorithm can be generated for each group 21, 22, or 23, and used by the monitoring devices within the respective groups to determine a status of a monitored patient. Thus, for twenty-six different devices, only three different algorithms need be determined whilst preserving a high degree of specificity and relevance of each algorithm (with respect to the monitoring devices). Thus, a processing power of calculating appropriate algorithms can be reduced without significantly affecting the relevance and/or efficiency of a particular algorithm.

In at least one embodiment, each medical characteristic associated with the data source may have a corresponding importance or priority (such as a numerical figure indicating a relative importance or priority of the characteristic).

The step 12 of clustering the data sources may depend upon the relative importance of the medical characteristics of each data source. By way of example, a particular data source having a first medical characteristic of high importance may be grouped into a set/group where a commonly shared characteristic is this first medical characteristic.

The importance may provide a weighting to the clustering of the data sources. By way of example, a numerical importance value of each available medical characteristic may be used when performing a k-means clustering algorithm a k-medoids clustering algorithm or any other similar clustering algorithm.

By way of example, each input vector $P_s$ associated with a data source may be represented by:

$$P_s = (i_1.p_1, i_2.p_2 \ldots i_N.p_N) \tag{4}$$

where p represents a (logical) availability of a different medical characteristic, i represents an importance value of a respective medical characteristic and N is a number of medical characteristics associated with the data source.

The importance may represent an importance, priority or purpose of the medical characteristic with respect to the data source, a patient, a patient monitoring device or medical trends. The type of the relative importance may vary depending upon the type of data source. Of course, more than one importance value may be provided each representing a different relative importance, and may each provide a respective weighting value.

The importance value may thereby be a numerical value representing a relative importance of the medical characteristic. The importance value may be a ratio, fraction (e.g. ranging from 0 to 1) or percentage indicating a perceived importance of the medical characteristic.

In other embodiments, the importance value is a categorical data point (e.g. High, Medium or Low). An algorithm may process the importance value, for example, assigning a numerical value to each relevant categorical data point.

In one scenario, where each data source represents a patient, medical characteristics relating to the heart may be more important for a patient with a known heart defect. Thus, an importance value for heart-related medical characteristics may be greater than other medical characteristics (such as weight).

In another scenario, where each data source represents a monitoring device, it is recognized that the main purpose of a pulse oximeter is to measure $SpO_2$, rather than heart rate, even though the pulse oximeter may measure both. Thus the relative importance of $SpO_2$ (when captured by a pulse oximeter) may be greater than the relative importance of heart rate (when captured by the same pulse oximeter).

Clustering data sources based on the relative importance may ensure that data sources having medical characteristics of a similar importance are grouped together. This increases a likelihood that an algorithm generated for a group uses the medical characteristics of a high importance, thereby improving a likelihood of a generated algorithm accurately identifying a status of a patient.

Table 2

| Characteristic | A | | B | | C | | D | |
|---|---|---|---|---|---|---|---|---|
| | p | i | p | i | p | i | p | i |
| 1 | Yes | High | Yes | High | No | --- | No | --- |
| 2 | Yes | Low | Yes | High | No | --- | No | --- |
| 3 | Yes | High | Yes | High | No | --- | No | --- |
| 4 | Yes | High | No | --- | Yes | Low | Yes | Low |
| 5 | Yes | High | No | --- | Yes | Low | Yes | Low |

(continued)

| Characteristic | A | | B | | C | | D | |
|---|---|---|---|---|---|---|---|---|
| | p | i | p | i | p | i | p | i |
| 6 | Yes | Low | No | --- | Yes | High | Yes | High |
| 7 | No | --- | No | --- | Yes | High | Yes | High |
| 8 | No | --- | No | --- | Yes | High | Yes | High |

Table 2 illustrates a second use-case scenario, being a modified version of the first use-case scenario. Each medical characteristic (A-D) associated with each device (1-8) has been assigned an importance value i. Here, the importance value is binary (High or Low), but in other embodiments the importance value is a scalar number or another representation.

The eight patient monitoring devices could be divided into three sets or groups based on a relative importance of the medical characteristics. A first group (Devices 1-3), each associated with characteristics A, B; a second group (Devices 4-5) each associated with characteristics A, B, C; and a third group (Devices 6-8) each associated with characteristics C, D.

In particular, it is clear that the importance of each characteristic is taken into account when performing clustering. In particular, devices associated with the same (highly important) characteristics are clustered into a same group.

In embodiments, the input data indicates how each medical characteristic of the data source is obtained and the clustering of the data sources may depend upon how the medical characteristics of that data source are obtained.

For example, the input data may indicate medical characteristics associated with monitoring devices (i.e. the data sources are monitoring devices) and the monitoring devices may be clustered into groups which obtain the same medical characteristics in a similar way.

This improves a consistency amongst the clustering of the data sources and improves a similarity of data sources in a same group. Thus, algorithms generated for a group of data sources may better represent or take into account the appropriate medical characteristics, improving the algorithm.

In some embodiments each medical characteristic is associated with a temporal availability or a historic availability. A temporal availability may indicate how often, how long or how regularly a value for that characteristic can be or has been (reliably or relevantly) obtained. The temporal availability may act as an indicator of the availability of a medical characteristic.

In preferably embodiments, the temporal availability may indicate an average frequency of a value of a medical characteristic being (reliably) sampled, measured or other obtained. This may take into account, for example, inconsistently spaced measurements of a particular medical characteristic.

In one example, a temporal availability of a heart rate could indicate that a heart rate can be reliably obtained once a second, whereas a temporal availability of a respiratory rate may indicate that the respiratory rate can only be reliably obtained once every 30 seconds. In another example, certain monitoring devices may be operable at different speeds or may only be periodically available, which may be indicated by the temporal availability.

The input vector $P_s$ representing each data source may be represented by:

$$P_s = (t_1, t_2 \dots t_N) \tag{5}$$

where t represents a temporal availability of a medical characteristic and N is a number of medical characteristics associated with the data source. The importance value i, described earlier, may optionally weight each temporal availability in some embodiments.

Thus a temporal availability may act as an availability of a medical characteristic, and replace a categorical or logical availability.

The temporal availability may be a relative temporal availability (e.g. when compared to other medical characteristics of the data source), such as a relative (average) frequency or period of sampling the medical characteristic. The temporal availability may be a ratio or normalized value.

In particular, the temporal availability of a medical characteristic of a patient may represent a proportion of times that said medical characteristic is measured when one or more measurements of medical characteristics of the patient is taken.

Measurements of a plurality of patient characteristics may be simultaneously taken at a plurality of different sampling times. The temporal availability of a patient characteristic may indicate what proportion, percentage or ratio of these different sampling times that a measurement of the given patient characteristic is obtained.

In one example, each data source is a patient associated with only two measurable medical characteristics (weight and heart rate). A weight of a first patient may only be measured in 50% of samples or observations of the first patient ($t_1$ = 0.5), whereas a heartrate is measured in 90% of samples of observations ($t_2$ = 0.9). A weight of a second patient may be measured in 100% of samples or observations of the second patient ($t_1$ = 1.0), whereas a heartrate is measured in 20% of samples of observations ($t_2$ = 0.2). The input vectors $P_s$ representing the first and second patients may thereby be (0.5, 0.9) and (1.0, 0.2) respectively.

Grouping based on temporal availability advantageously accounts for inconsistent patient monitoring, such as may be caused by patients not correctly or consistently monitoring patient characteristics or using monitoring devices or by faulty devices. Algorithms which rely on patient characteristics that are inconsistently monitored, e.g. relative to other characteristic, would significantly degrade performance of determining a patient status, as relevant up-to-date patient information may be unavailable. Taking a temporal availability into account when grouping data sources, and thereby in the process of generating an algorithm, mitigates an impact of a low temporal unavailability of a medical characteristic.

Thus, clustering data sources based on the temporal availability ensures that medical characteristics that are less available do exert a disproportionate influence of the clustering algorithm. This increases a likelihood that an algorithm generated for a group uses the medical characteristics which are more available, thereby improving a likelihood of a generated algorithm accurately identifying a status of a patient (e.g. using the most up-to-date information or more accurately reflecting a current patient status).

Moreover, clustering using temporal availabilities also helps ensure that more similar devices/patients are grouped together and improves an overall efficiency of any algorithm generated. In particular, the algorithm may more accurately represent the available characteristics to a patient at a given time.

The step of generating an algorithm for a group or cluster may take into account the temporal availability of medical characteristics associated with data sources in that group of cluster. For example, medical characteristics having an average (across the group) temporal availability below a predetermined number may be excised or removed from an algorithm. In other embodiments, an average temporal availability of a medical characteristic is used to weight a respective coefficient of an algorithm.

Embodiments may thereby improve an accuracy and precision of an algorithm and reduce the influence of potentially temporally unavailable medical characteristics (e.g. old or inaccurate data) on the algorithm.

In other embodiments, the temporal availability indicates whether a particular characteristic is currently active with respect to a data source, for example, whether a possibly measurable characteristic is currently available or active (even though it may be generally available). Clustering on such a basis may ensure that characteristics which are not currently active do not contribute to a grouping of the data sources, and therefore do not influence the generation of the algorithm. Thus, any generated algorithm may more accurately represent a given group or set of data sources.

Thus, the invention may distinguish between measurable characteristics that are missing incidentally, so as to be temporarily unavailable (e.g. due to non-compliance, device malfunction or connectivity issues) or systematically (i.e. never available).

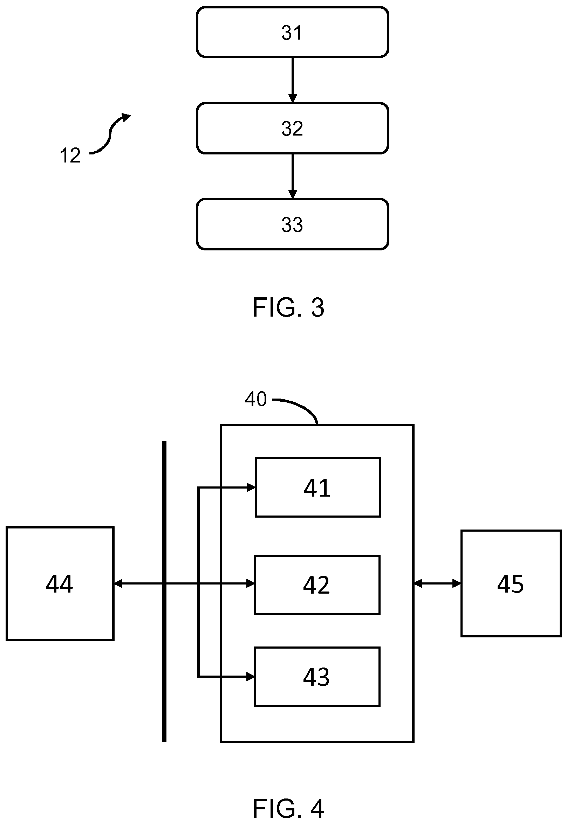

Figure 3 illustrates an embodiment of the step 12 of clustering the data sources.

The step 12 comprises a step 31 of applying a plurality of different clustering algorithms to the input data. In this way a respective plurality of clustering results are obtained, each associated with a different clustering algorithm. Each clustering result comprises all of the data sources clustered, grouped, divided or otherwise arranged into one or more (e.g. two or more) sets or groups.

The step 12 also comprises a step 32 of calculating a coverage value for each clustering result. The coverage value represents a percentage of the data sources (in a particular clustering result) that meet a predetermined criterion. In this way, a measure as to the effectiveness of a clustering (according to this predetermined criterion) can be determined.

The coverage value may provide an indication as to how many of the data sources have been clustered into a group sharing at least one same measurable medical characteristic. That is, the coverage value indicates a proportion of data sources that are arranged into a group for which a corresponding algorithm (of the group) would use medical characteristics that are associated with the patient or device.

The step 12 also comprises a step 33 selecting a clustering result based on the coverage value of each clustering result.

Thus, numerous attempts are made to cluster the input data into groups of data sources and the most appropriate clustering result (i.e. the most appropriate grouping or the most effective attempt) is selected for the purposes of generating an algorithm. This will improve a likelihood of a clustering being effective and there being an improved similarity between data sources in a group and a greater difference between different groups.

Different clustering algorithms may group the patient data into a different number of sets or groups. For example, each clustering algorithm may comprise a k-means clustering process or k-medoids clustering algorithm where the value for k is altered for each different clustering algorithm.

Each different clustering algorithm may thereby be a single or same clustering method or technique (e.g.

applying a k-means algorithm) which is able to output different clustering results (e.g. dependent upon a characteristic of the clustering algorithm, a number of times an algorithm is applied, a number of iterations of the algorithm and so on). In other embodiments, different clustering algorithms represent different clustering methods or techniques (e.g. a k-means clustering method and a biclustering method). Of course, the different clustering algorithms may contain a mix of such concepts.

In some embodiments, only a single algorithm is applied, where the output of this algorithm can comprise any number of clusters. The total number of clusters or groups of data sources can range from 1 to the number data sources, depending upon the algorithms and characteristics or variables of such algorithms.

The total number of clusters or groups of data sources may be user defined. Preferably, the total number of groups or clusters is no more than 5. For the sake of efficiency, it has been recognized that it is preferred for an algorithm to be optimized, generated or prepared for no less than 20% of the data sources (i.e. one fifth or 20% of the total number). Thus, it is preferably for there to be no more than 5 groups of data sources, for which respective algorithms may be generated.

The step 33 of selecting a clustering result may comprise selecting the clustering result having the highest or greatest coverage value.

In another embodiment, the step 33 may comprise selecting the clustering result having the highest ratio of coverage value to number of groups of that clustering result. In particular the coverage value of each clustering result may be divided by the number of groups in that clustering result, to obtain a ratio value. The clustering result having the highest ratio value is selected. This may provide a more efficient method of identifying an appropriate clustering result, resulting in a reduced workload when generating one or more algorithms.

In yet another embodiment, the step 33 may comprise selecting the clustering result having the lowest number of groups amongst those clustering results that have a coverage value higher/greater than a predetermined coverage value (e.g. 60% or 80%). Thus a minimum coverage value is assured whilst choosing the most efficient grouping. This further reduces a workload for generating algorithms for the patient data whilst ensuring appropriate algorithms are generated.

Thus, the most applicable clustering result may be determined in different ways. In preferable examples, the selected clustering result is that providing the lowest number of groups whilst maintained a coverage value above a predetermined value (e.g. at least 80%).

The predetermined coverage value may be around 80%, such that 80% of the data sources must meet the predetermined criterion to adhere the predetermined coverage value. Other coverage values will be appreciated by the skilled person, for example, around 40%, 50%, 70% or 90%. In some embodiments, the predetermined coverage value is determined or input by a user.

The coverage value may be the proportion of data sources that are associated with medical characteristics shared by at least one or all data sources in a respective group of the data source.

Thus, a data source may meet the predetermined criterion if the data source is associated with the medical characteristics that are common in the group of said data source. Thus, if the most common medical characteristic(s) of a group is associated with a data source of that group, the data source may meet the predetermined criterion.

In another embodiment, the data source may meet the predetermined criterion if a distance between the input vector $P_s$ of the data source and the average vector of the group of data sources (being an average of all input vectors of the group) is less than a predetermined value.

Thus, in some examples, the coverage value indicates a proportion, ratio or percentage of the data sources in a group that are represented by an input vector $P_S$ which is less than a predetermined distance from an average input vector of the group. By way of example, in a k-medoids or k-means clustering algorithm, a cluster center is automatically determined. This cluster center may define or represent the (average) input vector of the group to be used in such a determination.

In some embodiments, the cluster center (representing the average input vector) may be used as the basis for calculating an algorithm for the associated group of data sources. Thus, the average input vector of the group of data sources may be used to generate an optimized algorithm which fairly represents each data source that group.

In another embodiment, where the input data further indicates, for each data source, a importance value of each measurable medical characteristic associated with said data source, a data source meets the predetermined criterion if at least one available measurable medical characteristics for said data source, which has a importance value equal to or above a predetermined importance value, is common to more than a predetermined proportion of the data sources in the same group as the said data source.

Thus, a data source may only meet the predetermined criterion if a medical characteristic of that data source, having an importance value greater than a predetermined value, is shared by more than a predetermined portion (e.g. 60%, 80% or 100%) of all data sources in the group of that data source.

Where the importance value is a numerical value between 0 and 1, the predetermined importance value may be at least 0.6, for example, at least 0.8. In other embodiments, the predetermined importance value may be an importance value that indicates a 'High' importance of a medical characteristic.

In some embodiments, the data source only meets the predetermined criterion if all medical characteristics of that data source, having an importance value greater than a predetermined value, are shared by more than a predetermined portion (e.g. 60%, 80% or 100%) of all data sources in the group of that data source.

Proposed embodiments thereby ensure that important characteristics of a data source are used to assess which clustering result to select. This will help prevent algorithms from being generated that do not take into account potentially vital medical characteristics for determining a patient's status.

In yet another embodiment, the step 12 of clustering comprises applying a plurality of different clustering algorithms to the input data to obtain a respective plurality of clustering results, each clustering result comprising all of the data sources clustered into one or more groups; calculating a distinctiveness score for each clustering result, the distinctiveness score indicating a distinctiveness of the groups within a respective clustering result; and selecting a clustering result based on the distinctiveness score of each clustering result.

Thus, rather than calculating a coverage score to determine which clustering result to select, a distinctiveness score of the groupings may be determined. The distinctiveness score may be any known distinctiveness score or validity index of the clustering. This may include, for example, a Dunn index, a Davies-Bouldin index or a Silhouette index (e.g. average silhouette width). Any number of clustering results may be obtained, as previously described.

This embodiment provides an advantage of ensuring that groups are well spaced and distinctive, such that it can be assumed that any algorithm generated for a group is well optimized to that group, and that different algorithms have been optimized appropriately.

In some embodiments, the step 12 of clustering comprises presenting a user with a visual representation of the input data or an output of a processed input data. For example, the user may be presented with a dendrogram which illustrates a similarity, proximity or relationship between different data sources of the input data.

The step of clustering may comprise receiving a user input indicating the groups of data sources or how the data sources are to be grouped. For example, a user may directly indicate which data sources are to be grouped together. In another example, a user may select, modify or create a clustering algorithm to be performed on the input data. Thus, the user may be able to select, alter, modify or add groups of data sources based on the visual representation of the input data. The step of clustering may thereby comprise receiving a user input indicating the groups of data sources, thereby providing a user with a free choice of selecting a clustering algorithm.

In some embodiments, a plurality of different clustering results are presented to a user, and the step of clustering comprises receiving a user input indicating which clustering result to select. Thus, rather than determining a coverage value of distinctiveness score, a user input may determine a clustering result.

Such embodiments improve a customizability of the method for determining a status of the patient, and may allow for new patterns or similarities to be identified. A user may be able to use their clinical expertise, novel hypotheses, or experimental understandings to identify potential groups of data sources.

In some embodiments, the method is adapted to comprise a step of displaying additional information to identify patterns. For example, where the data sources are monitoring devices, the method may display additional information about a monitoring device such as the number of patients associated with that device, a length of time a device has been in use, a number of monitoring devices used at a location and so on. This may reduce a burden of a clinician in manually retrieving such information.

Of course, the assessment of the input data may be performed automatically, for example, using a machine-learning algorithm or any method previously described (e.g. using a clustering algorithm to automatically determine groups of data sources as previously described).

Figure 4 illustrates a processor arrangement 40 according to an embodiment of the invention.

The processor arrangement 40 comprises a data receiving unit 41 adapted to receive input data which indicates measurable medical characteristics for each of a plurality of data sources.

The processor arrangement 40 also comprises a clustering unit 42 adapted to cluster, using the input data, the data sources into one or more groups based on the measurable medical characteristic for each of the data sources. Preferably, the clustering unit 42 is adapted to cluster, group, or otherwise arrange the data sources into at least two sets, groups or clusters.

The processor arrangement 40 also comprises a calculating unit 43 adapted to calculate, for each of one or more groups of data sources, an algorithm for determining a status of a patient based on the measurable medical characteristics for the respective group of data sources.

The processor arrangement may be adapted to communicate with a memory system 44, for example, using a bus. The memory system may, for example, store the input data, the algorithms generated by the calculating unit or historic/default algorithms for modification by the clustering unit.

The processor arrangement 40 may be adapted to communicate with a user interface 45, for example, to display a visual representation of the input data or to display a clustering result. The user interface 45 may be adapted to receive a user input, as previously described.

The various elements of the processor arrangement 40 may be adapted, where appropriate, to perform the steps of any method previously described.

As discussed above, embodiments make use of a processor arrangement. The processor arrangement can be implemented in numerous ways, with software and/or hardware, to perform the various functions or steps required. A processor is one example of a processor arrangement which employs one or more microprocessors that may be programmed using software (e.g., microcode) to perform the required functions. A processor arrangement may however be implemented with or without employing a processor, and also may be implemented as a combination of dedicated hardware to perform some functions and a processor (e.g., one or more programmed microprocessors and associated circuitry) to perform other functions.

Examples of processor arrangement components that may be employed in various embodiments of the present disclosure include, but are not limited to, conventional microprocessors, application specific integrated circuits (ASICs), and field-programmable gate arrays (FPGAs).

In various implementations, a processor arrangement may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

There may therefore be provided a computer program product comprising a computer readable storage medium having computer readable program instructions embodied therewith to, when executed on a processor arrangement, cause said processor arrangement to implement any method previously described.

A medical characteristic is commonly referred to as a sign, symptom or observation of patient that may be relevant to their overall health. These may include, amongst others: pulse rate, respiratory rate, respiratory volume, tidal volume, systolic blood pressure, diastolic blood pressure, overall blood pressure, body weight, clinical observations, skin tone, patient alertness or responsiveness, blood sugar level, hemoglobin level, white blood cell count, body temperature, oxygen saturation ($SpO_2$), Glasgow Coma Scale score, prognostic signs, anamnestic signs, diagnostic signs, pathognomic signs, x-ray analysis results, CAT scanner analysis results, and so on.

The data sources of the input data may be a same type of data source. Thus, each data source of a same input data may represent only one of a patient, patient monitoring device, diagnosis or treatment option.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A method (1) of generating at least one algorithm for determining a status of a patient, the method comprising:

   receiving (11) input data which indicates measurable medical characteristics associated with each of a plurality of data sources;
   clustering (12), using the input data, the data sources into one or more groups based on the measurable medical characteristic for each of the data sources; and
   calculating (13), for each of one or more groups of data sources, an algorithm for determining a status of a patient based on the measurable medical characteristics for the respective group of data sources.

2. The method of claim 1, wherein each data source represents one of: a patient; a patient monitoring device; a diagnosis; and a treatment option.

3. The method of any preceding claim, wherein:

   the input data further indicates, for each data source, a importance value of each measurable medical characteristic associated with said data source; and
   wherein the clustering, using the input data, is performed based on the importance values of the measurable medical characteristics associated with each data source.

4. The method of any preceding claim, wherein:

the input data further indicates, for each data source, a temporal availability of each measurable medical characteristic associated with said data source; and

the clustering, using the input data, is performed based on the temporal availability of the measurable medical characteristics associated with each data source.

5. The method of any preceding claim, wherein the step (12) of clustering comprises:

applying (31) a plurality of different clustering algorithms to the input data to obtain a respective plurality of clustering results, each clustering result comprising all data sources clustered into one or more groups;

calculating (32) a coverage value for each clustering result, the coverage value indicating a percentage of data sources meeting a predetermined criterion; and

selecting (33) a clustering result based on the coverage value of each clustering result.

6. The method of claim 5, wherein the step of selecting the clustering result comprises selecting the clustering result which is associated with one or more of:

the greatest coverage value;

the greatest value resulting from dividing the coverage value by the number of groups in the clustering result; and

the clustering result having the lowest number of groups amongst the clustering results having a coverage value greater than a predetermined coverage value.

7. The method of claim 5 or 6, wherein a data source meets the predetermined criterion if the measurable medical characteristics associated with said data source comprise the most common measurable medical characteristic of all data sources in the same group as the said data source.

8. The method of claim 5 or 6, wherein:

the input data further indicates, for each data source, a importance value of each measurable medical characteristic associated with said data source; and

a data source meets the predetermined criterion if at least one available measurable medical characteristics for said data source, which has a importance value equal to or above a predetermined importance value, is common to more than a predetermined proportion of the data sources in the same group as the said data source.

9. The method of claim 4 or 5, wherein:

the input data further indicates, for each data source, a importance value of each measurable medical characteristic associated with data source; and

a data source meets the predetermined criterion if all measurable medical characteristics for the said data source, which have a importance value equal to or above a predetermined importance value, are common to more than a predetermined proportion of the measurable medical characteristics in the same group as the said data source.

10. The method of any of claims 1 to 3, wherein the clustering comprises:

applying a plurality of different clustering algorithms to the input data to obtain a respective plurality of clustering results, each clustering result comprising all of the data sources clustered into one or more groups;

calculating a distinctiveness score for each clustering result, the distinctiveness score indicating a distinctiveness of the groups within a respective clustering result; and

selecting a clustering result based on the distinctiveness score of each clustering result.

11. The method of any preceding claim, wherein the step of calculating an algorithm for a group of data sources is based on at least the most common measurable medical characteristic within the group of data sources.

12. A computer program product comprising a computer readable storage medium having computer readable program instructions embodied therewith to, when executed on a processor arrangement, cause said processor arrangement to implement the method of any preceding claim.

13. A processor arrangement (40) for generating at least one algorithm for determining a status of one or more patients, the processor arrangement comprising:

a data receiving unit (41) adapted to receive input data which indicates measurable medical characteristics for each of a plurality of data sources;
a clustering unit (42) adapted to cluster, using the input data, the data sources into one or more groups based on the measurable medical characteristic for each of the data sources; and
a calculating unit (43) adapted to calculate, for each of one or more groups of data sources, an algorithm for determining a status of a patient based on the measurable medical characteristics for the respective group of data sources.

14. The processor arrangement of claim 13, wherein:

the input data further indicates, for each of the data sources, a importance value of each measurable medical characteristic associated with said data source, and

the clustering unit is adapted to cluster, using the input data, based on the importance values of the measurable medical characteristics associated with each of the data sources.

15. The processor arrangement of any of claims 13 or 14, wherein the clustering unit is adapted to:

apply a plurality of different clustering algorithms to the input data to obtain a respective plurality of clustering results, each clustering result comprising all of the data sources clustered into one or more groups;
calculate a coverage value for each clustering result, the coverage value indicating a percentage of data sources meeting a predetermined criterion; and
select a clustering result based on the coverage value of each clustering result.

FIG. 1

FIG. 2

31

32

12

33

FIG. 3

40

41

44      42      45

43

FIG. 4

# EP 3 460 808 A1

**European Patent Office**
Europäisches Patentamt
European Patent Office
Office européen des brevets

## EUROPEAN SEARCH REPORT

**Application Number**

EP 17 19 2399

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/095141 A1 (SAREL ODED [IL]) 26 August 2010 (2010-08-26) * abstract * * page 7, line 25 * * page 24, line 11 - line 21 * * figure 12 * | 1-15 | INV. G16H50/70 |
| X | US 2015/059756 A1 (BARONOV DIMITAR V [US] ET AL) 5 March 2015 (2015-03-05) * paragraph [0003] * * paragraph [0023] - paragraph [0024] * | 1-15 | |

**TECHNICAL FIELDS SEARCHED (IPC)**

G16H

The present search report has been drawn up for all claims

| Place of search | Date of completion of the search | Examiner |
|---|---|---|
| Munich | 5 March 2018 | Wittke, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

19

**ANNEX TO THE EUROPEAN SEARCH REPORT
ON EUROPEAN PATENT APPLICATION NO.**

EP 17 19 2399

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-2018

| Patent document cited in search report | Publication date | Patent family member(s) | Publication date |
|---|---|---|---|
| WO 2010095141 A1 | 26-08-2010 | CN 102422290 A<br>US 2010217736 A1<br>WO 2010095141 A1 | 18-04-2012<br>26-08-2010<br>26-08-2010 |
| US 2015059756 A1 | 05-03-2015 | US 2013054264 A1<br>US 2013085775 A1<br>US 2015059756 A1<br>WO 2012122096 A2 | 28-02-2013<br>04-04-2013<br>05-03-2015<br>13-09-2012 |

EPO FORM P0459

For more details about this annex : see Official Journal of the European Patent Office, No. 12/82